# EUROPEAN PATENT SPECIFICATION

(11) **EP 2 809 272 B1**
(45) Date of publication and mention of the grant of the patent: **04.01.2017**
(21) Application number: 13743728.1
(22) Date of filing: 01.02.2013
(51) Int. Cl.: A61F 2/24

(54) **INVERTIBLE TISSUE VALVE**
UMKEHRBARE GEWEBEKLAPPE
VALVULE TISSULAIRE RÉVERSIBLE

(30) Priority: 01.02.2012 US 201261593817 P
(43) Date of publication of application: 10.12.2014
(73) Proprietor: Hlt, Inc., Maple Grove, MN 55369 (US)
(72) Inventor: GAINOR, John P., Mendota Heights, MN 55118 (US)
(74) Representative: KIPA AB
(86) International application number: PCT/US2013/024514
(87) International publication number: WO 2013/116785

(56) References cited:
- WO-A1-2005/058408
- WO-A1-2006/036690
- WO-A1-2006/083763
- WO-A1-2008/072838
- WO-A1-2008/097589
- WO-A1-2009/153768
- WO-A2-2011/143263
- US-A1- 2003 014 104
- US-A1- 2004 093 075
- US-A1- 2008 195 199
- US-A1- 2008 300 678
- US-A1- 2011 276 128

## Description

### BACKGROUND OF THE INVENTION

Replacing heart valves with prosthetic valves was, until recently, a complicated surgical procedure that involved cutting open the chest, establishing blood flow through a blood pump, stopping the heart, etc. This complicated procedure, even when performed perfectly, required extensive recovery time due to the invasiveness and damage done to access the implantation site. Additionally, the risk of infection or other complications is extremely high.

Numerous advancements have been made to develop prosthetic valves that can be implanted percutaneously, using a catheter to snake the prosthetic valve through the vasculature to the implantation site. If successful, the recovery time is greatly minimized relative to conventional open-heart surgery.

A designer of a percutaneously-delivered prosthetic valve is faced with numerous challenges, however. First and foremost is designing a prosthetic valve that can be compressed enough to be inserted into a catheter small enough to be navigated to the valve site through the vasculature. Other challenges include anchoring the valve at the valve site so the valve does not migrate after release; including a support structure for the valve that is robust enough to push the native, often calcified valve out of the way and prevent it from later interfering with the function of the new valve; ensuring that the new valve allows proper flow in a desired direction and effectively stops flow in the opposite direction; ensuring that no blood flows around the sides of the implanted device (this is known as perivalvular leakage); designing a prosthetic valve device that does not fail due to fatigue after hundreds of thousands of cycles of leaflet function; designing a valve that meets all of these criteria and can still be manufactured economically; and the list goes on.

These prosthetic valves, and their respective delivery catheters, are designed to replace a particular native valve. One native valve that presents unique challenges is the aortic valve. The aortic valve controls blood flow from the left ventricle into the aorta. Reaching the aortic valve percutaneously is typically accomplished using one of two approaches.

The first approach is a transfemoral retrograde approach whereby the femoral artery is accessed near the groin of the patient, and followed upstream to the aortic valve. The transfemoral approach is retrograde because travel is against the flow of blood and thus the downstream side of the aortic valve is reached first.

The second approach is a transapical antegrade approach which can be performed via a left anterolateral minithoracotomy. This approach punctures the apex of the heart to provide direct access to the left ventricle. Thus, the aortic valve is approached from the upstream (antegrade) side.

There are advantages and disadvantages to both approaches. The transfemoral approach is considered less invasive because the heart is not punctured. However, the navigation is much longer and access to the aortic valve requires the necessarily longer delivery catheter to follow the curve of the aortic arch at the end of the path to the valve. Thus, the delivery catheter must be more maneuverable and the prosthetic valve must not interfere with the maneuverability of the catheter while the valve is loaded into it. The transapical approach requires puncturing the myocardial sac and the apex of the heart and traversing the chest cavity. In each of these cases, the heart is beating as well, adding more difficulty to the procedure.

WO2008097589 discloses an apparatus that includes a valve having a valve frame, a valve leaflet coupled to the valve frame, and a leaflet transition member coupled to the valve leaflet.

WO2009153768 an esophageal valve comprising a polymeric valve body having an outer support region at least three valve leaflets, and a main body region extending between the support region and the valve leaflets.

### OBJECTS AND SUMMARY OF THE INVENTION

The invention is defined in the claims.

One aspect is directed to a prosthetic valve device for use in replacing a native aortic valve using a retrograde approach.

Another aspect is directed to a prosthetic valve device that is sized to replace an aortic valve and capable of being delivered using a small, flexible catheter.

Another aspect is directed to a prosthetic valve device that comprises two components positioned in series (spaced apart axially) in a delivery catheter to reduce the size of the delivery catheter required.

Another aspect is directed to a prosthetic valve device that comprises two components positioned in series during navigation whereby the two components can be located together upon delivery to the target site.

One aspect provides a device for replacing a native valve comprising: a stent; a tissue sleeve; and, an anchoring mechanism usable to secure said tissue sleeve within said stent; wherein, in a configuration inside a delivery catheter, said anchoring mechanism is not located within said stent; and wherein, in a deployed configuration, said tissue sleeve is located within said stent.

Another aspect provides that said tissue sleeve is inverted (i.e. inside-out) relative to said configuration inside said delivery catheter.

Another aspect provides that the tissue sleeve is connected to said stent at a first end and connected to said anchoring mechanism at a second end, opposite said first end.

Another aspect provides an anchoring mechanism that comprises a wireform.

Another aspect provides an anchoring mechanism that comprises a ring.

Another aspect provides an anchoring mechanism that comprises a ring and a wireform.

Another aspect provides an anchoring mechanism that comprises a ring attached to a first end of said tissue sleeve and a second ring attached to an opposite side of said tissue sleeve.

Another aspect provides a tissue sleeve that comprises valve leaflets.

Another aspect provides a tissue sleeve that comprises pinch points that result in the formation of valve leaflets when implanted.

Another aspect provides a method for replacing a native blood valve comprising: expanding a stent within said native valve; anchoring a tissue sleeve at a proximal end of said stent; advancing said tissue sleeve into said stent, resulting in an inversion of said tissue sleeve; and allowing blood to flow through said tissue sleeve in a proximal direction while not allowing blood to flow through said tissue sleeve in a distal direction.

In one aspect a method is provided wherein expanding a stent within said native valve comprises expanding a stent within said native valve prior to introducing said tissue sleeve at a proximal end of said stent.

In another aspect a method is provided wherein anchoring a tissue sleeve at a proximal end of said stent occurs prior to the step of expanding a stent within said native valve.

In another aspect a method is provided wherein anchoring a tissue sleeve at a proximal end of said stent comprises attaching said tissue sleeve to said proximal end of said stent.

In another aspect a method is provided wherein anchoring a tissue sleeve at a proximal end of said stent comprises allowing a ring to expand at a proximal end of said stent, said ring attached to said tissue sleeve.

In another aspect a method is provided wherein allowing blood to flow through said tissue sleeve in a proximal direction while not allowing blood to flow through said tissue sleeve in a distal direction comprises providing valve leaflets attached to said tissue sleeve such that said valve leaflets are located on an outside surface of said tissue sleeve prior to said inversion and on an inside of said tissue sleeve after inversion.

In another aspect a method is provided wherein allowing blood to flow through said tissue sleeve in a proximal direction while not allowing blood to flow through said tissue sleeve in a distal direction comprises expanding a wireform within said tissue sleeve, said wireform creating valve leaflets in said tissue sleeve when expanded.

In another aspect a method is provided wherein allowing blood to flow through said tissue sleeve in a proximal direction while not allowing blood to flow through said tissue sleeve in a distal direction comprises providing pinch points in a proximal end of said tissue sleeve, after said tissue sleeve is inverted, said pinch point causing blood flow to form valve leaflets out of said tissue sleeve when blood is flowing in a distal direction.

In another aspect a method is provided further comprising the step of anchoring said tissue sleeve near a distal end of said stent after the step of advancing said tissue sleeve into said stent.

One aspect provides a valve assembly for implantation within a stent comprising: a tissue sleeve; at least one anchoring mechanism for securing said tissue sleeve within said stent; wherein said tissue sleeve is connected to said at least one anchoring mechanism.

Another aspect provides a valve assembly wherein said tissue sleeve is attached to a proximal end of said stent.

Another aspect provides a valve assembly wherein said at least one anchoring mechanism comprises a wireform.

Another aspect provides a valve assembly wherein said at least one anchoring mechanism comprises at least one expandable ring.

Another aspect provides a valve assembly wherein said at least one expandable ring comprises a first expandable ring at one end of said tissue sleeve and a second expandable ring at another end of said tissue sleeve.

Another aspect provides a valve assembly wherein said tissue sleeve comprises valve leaflets.

Another aspect provides a valve assembly wherein said tissue sleeve forms valve leaflets when said at least one anchoring mechanism is expanded.

Another aspect provides a valve assembly wherein said tissue sleeve comprises valve leaflets that become function after said tissue sleeve is inverted.

### BRIEF DESCRIPTION OF THE DRAWINGS

These and other aspects, features and advantages of which embodiments of the invention are capable of will be apparent and elucidated from the following description of embodiments of the present invention, reference being made to the accompanying drawings, in which

- Figure 1: is a side view of an embodiment;
- Figures 2A-2D: illustrate a delivery sequence for the embodiment of Figure 1;
- Figures 3A-F: illustrate a delivery sequence for an embodiment;
- Figure 4: is a side view of an embodiment;
- Figure 5: is a side view of an embodiment;
- Figure 6: is a side view of an embodiment;
- Figures 7A-D: illustrate a delivery sequence for an embodiment; and
- Figures 8A-D: illustrate a delivery sequence for an embodiment.

### DESCRIPTION OF EMBODIMENTS

Specific embodiments of the invention will now be described with reference to the accompanying drawings. This invention may, however, be embodied in many different forms and should not be construed as limited to the embodiments set forth herein; rather, these embodiments are provided so that this disclosure will be thorough and complete, and will fully convey the scope of the invention to those skilled in the art. The terminology used in the detailed description of the embodiments illustrated in the accompanying drawings is not intended to be limiting of the invention. In the drawings, like numbers refer to like elements.

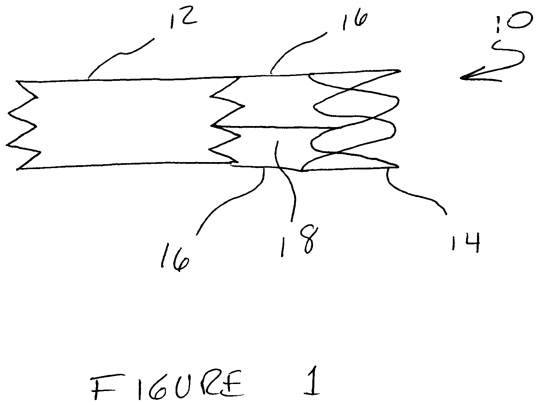

Referring first to Figure 1 there is shown a device 10. Device 10 generally includes a stent 12 connected to a valve assembly that includes a valve frame 14 and tissue connectors 16. Tissue 18 forms a prosthetic valve, shaped by the valve frame 14. The tissue connectors act as a stop when the valve frame 14 is advanced into the stent 12 during delivery. Note that the tissue 18 between the stent 12 and the valve frame 14 prevents blood from flowing around the valve frame 14. Thus perivalvular leakage is avoided.

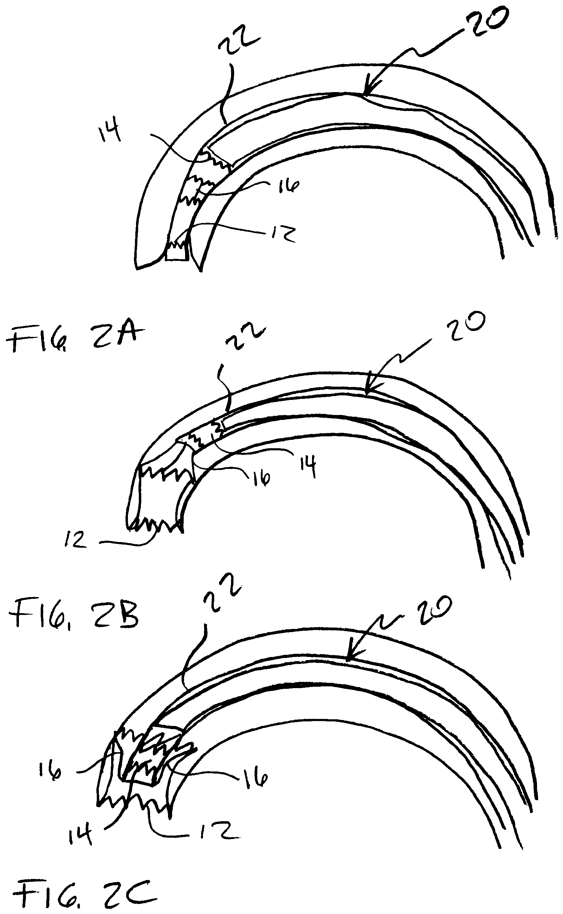

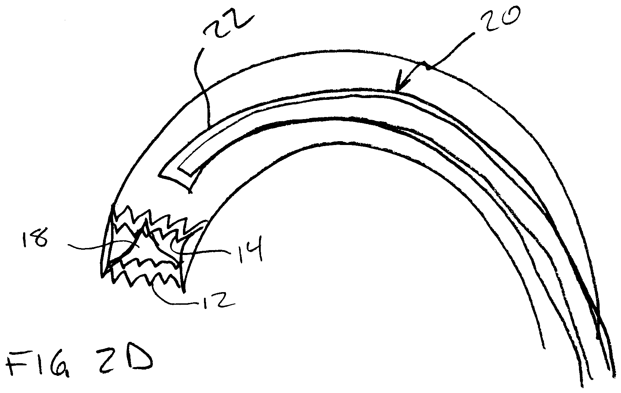

Thus, delivery of the device 10, as illustrated in Figures 2A-2D would involve navigation a catheter 20 to the valve site (Figure 2A), retracting a restraining sheath 22 until the stent 12 is released and allowed to expand (Figure 2B), advancing the still-constrained valve frame 14 into the expanded stent 12 until the tissue connectors 16 prevent further distal movement (Figure 2C), retracting the restraining sheath 22 until the valve frame 14 is released, allowing the valve frame 14 to expand within the stent 12 (Fig 2D).

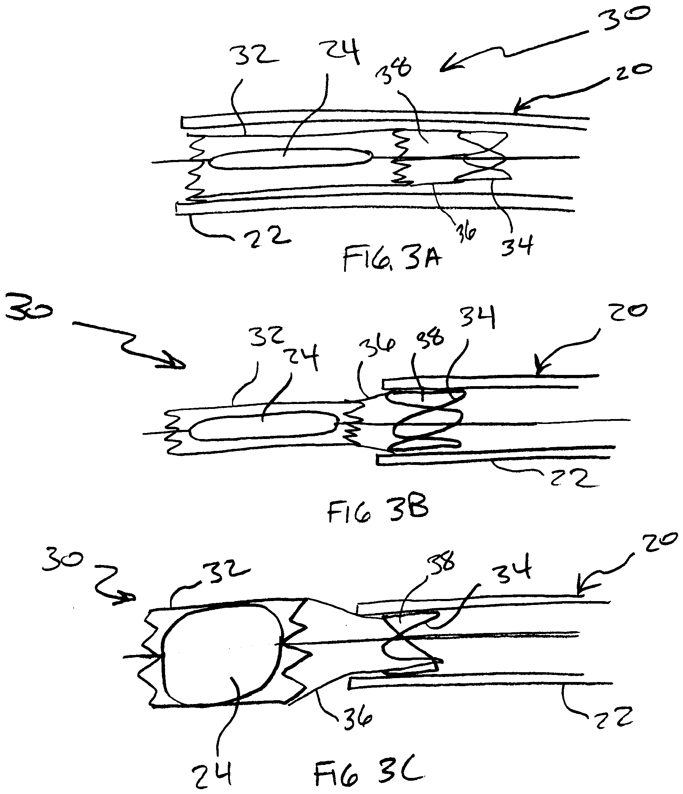

Figures 3A-3E illustrate an embodiment 30 wherein the stent 32 is balloon-expandable. Device 30 generally includes a stent 32 connected to a valve frame 34 with tissue connectors 36. Tissue 38 forms a prosthetic valve, shaped by the valve frame 34. The tissue connectors act as a stop when the valve frame 34 is advanced into the stent 32 during delivery. Note that the tissue 38 between the stent 32 and the valve frame 34 prevents blood from flowing around the valve frame 34. Thus perivalvular leakage is avoided.

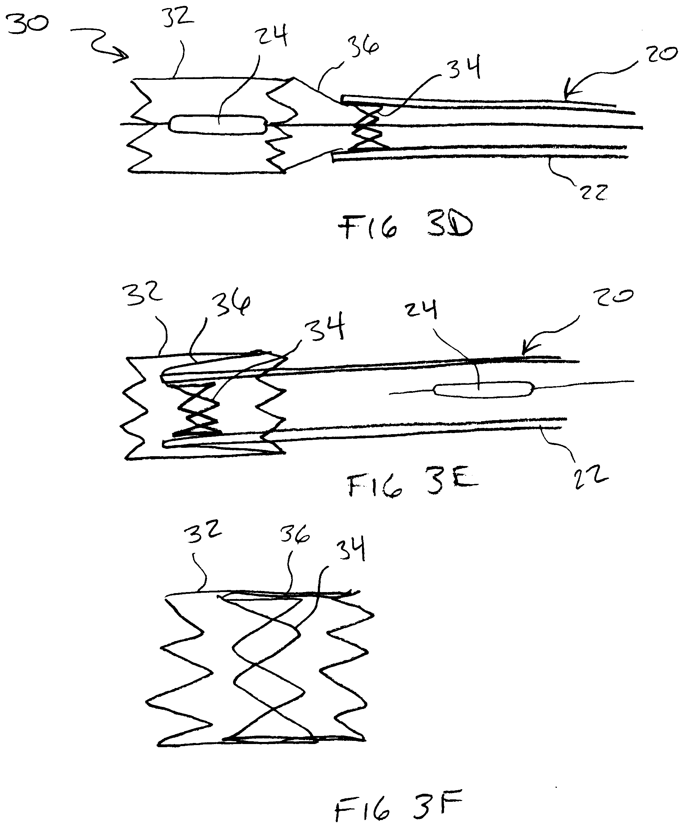

Thus, delivery of the device 10, as illustrated in Figures 3A-3E would involve navigation a catheter 20 to the valve site (Figure 3A), retracting a restraining sheath 22 until the stent 32 is released (Figure 3B), inflating a balloon 24 within the stent 32 to expand the stent 32 (Figure 3C), deflating the balloon 24 (Figure 3D), advancing the still-constrained valve frame 34 into the expanded stent 32 until the tissue connectors 36 prevent further distal movement (Figure 3E), retracting the restraining sheath 22 until the valve frame 14 is released, allowing the valve frame 14 to expand within the stent 12 (Fig 3F).

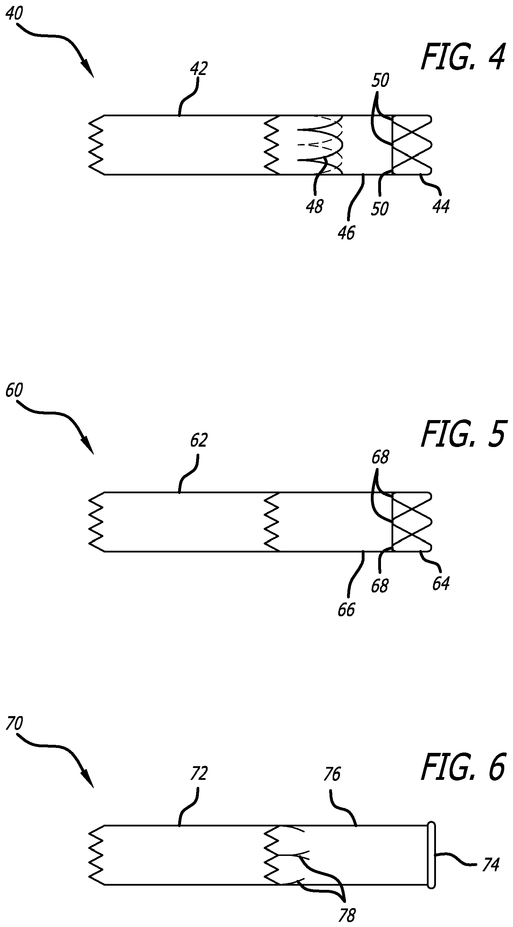

Figure 4 shows an embodiment 40 of a device. The device 40 shown in figure 4 Device 40 includes a stent 42, and a valve assembly that includes a wireform 44 and tissue 46. Rather than sewing the tissue to the wireform 44, thereby creating a valve frame for the prosthetic valve, the tissue 46 is attached to the wireform 44 at attachment points 50. Valve leaflets 48 are incorporated into the tissue 46 spanning between the stent 42 and the wireform 44. In the delivery configuration shown in Figure 4, the valve leaflets are located on the outside of the tissue sleeve 46. During delivery, using one of the procedures described above, as the wireform 44 is inserted inside the expanded stent 42, the tissue 46 and the leaflets 48 are inverted (i.e. turned inside-out) so that the valve leaflets are on the inside of the tissue sleeve 46. The wireform 44 is then expanded against the inside of the tissue sleeve 46, and aligned with the valve leaflets 48 so as not to interfere with their function. Thus the tissue sleeve 46, leaflets 48, and wireform 44, together form the prosthetic valve.

Device 40 allows a prosthetic valve to be formed using significantly less tissue material, as there is no need for two layers of tissue around the perimeter of the device after implant. Additionally, device 40 makes it possible to establish flow regulation through the device even at the intermediate stage of device implant.

Figure 5 shows a device 60 that is similar to that of Figure 4 except that it does not include valve leaflets. The device 60 includes a stent 62, and a valve assembly that includes a wireform 64, and a tissue sleeve 66. Tissue sleeve 66 is simply a tube of tissue. Once the device 60 is delivered, using any of the methods described above, the tissue sleeve 66 is inverted inside the stent 62 and the wireform 64 is expanded. When the wireform 64 is expanded inside the tissue sleeve 66, the wireform 64 creates leaflets due to the shape of the wireform 64. In this way, alignment of the wireform 64 inside the tissue sleeve 66 is not as critical as the embodiment 50 described above. Rather, one would merely need to ensure that there is enough tissue in the tissue sleeve 66 to effect coaptation of the resulting valve leaflets.

Figure 6 shows a device 70 that avoids the use of a wireform. The device 70 of figure 6 generally includes a stent 72 and a valve assembly that includes an anchor ring 74 and a tissue sleeve 76 connecting the stent 72 and the anchor ring 74. Upon deployment using any of the above methods, the stent 72 is expanded, the ring 74 is advanced into the stent 72, inverting the tissue sleeve 76, and the ring 74 is expanded. Pinch points 78 are formed in the tissue sleeve 76. The pinch points 78 create the formation of valve leaflets once the device is inverted and subjected to blood flow.

All of the devices heretofore described have been directed to designs that allow the device to be delivered in an axially, displaced, unassembled form and inverted and located upon delivery to create a finished device. These devices are thus directed toward a goal of being able to compress the devices into a small catheter, such as a 14 French catheter, for delivery. Potentially, however, areas where two components connect, such as the connection between the tissue sleeve and the stent, will have slight overlap that may result in additional thickness. Thus, the remaining embodiments described herein are directed to devices having stents and valves that are not connected to each other while they are inside the delivery catheter.

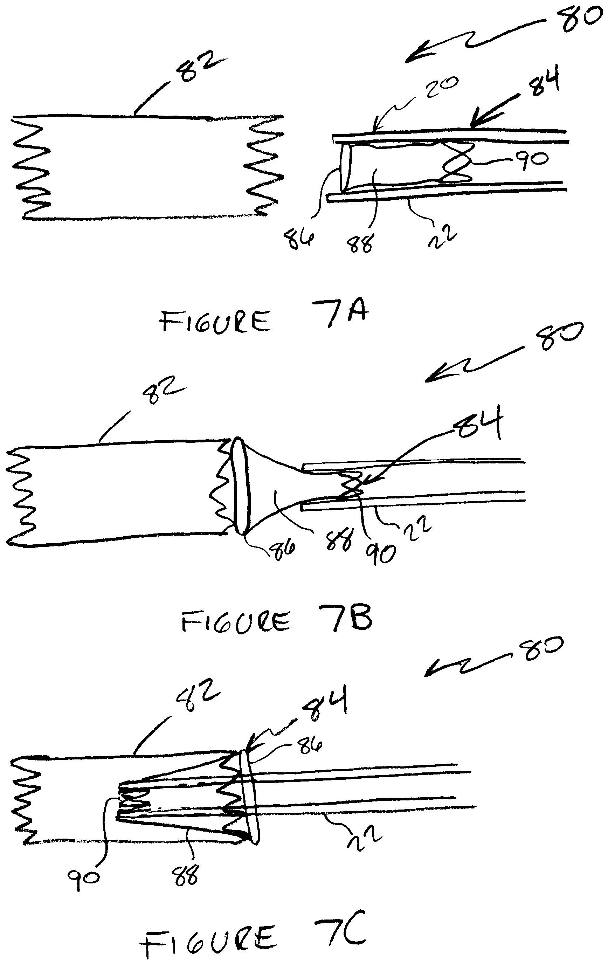

For example, referring to Figures 7A-D, there is shown a device 80 comprising two separate components: a stent 82 and a valve assembly 84. The valve assembly 84 includes a self-expanding anchor ring 86, a tissue sleeve 88 and a wireform 90. The anchor ring 86 anchors the valve assembly 84 in place until the wireform 90 is delivered. The anchor ring 86 also ensures proper reverse flow into the valve to effect coaptation of the resulting leaflets.

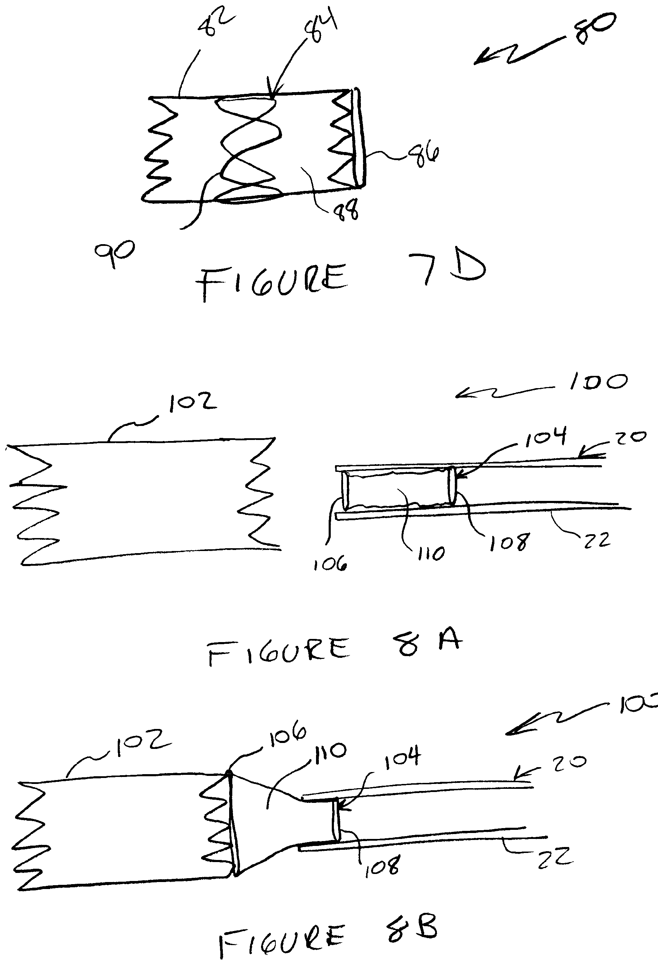

The delivery sequence for device 80 is as follows: As seen in Figure 7A, a stent 82 has been placed at the native valve site. The delivery catheter 20 is advanced until the distal end of the catheter is near of the proximal end of the stent 82. The sheath 22 of the delivery catheter 20 is then retracted releasing the ring 86. (Figure 7B) The ring expands just outside, or just inside of the stent 82. The delivery catheter 22 is advanced into the stent 82, causing the tissue sleeve 88 to invert. (Figure 7C) The wireform 90 is then released from the sheath 22 and allowed to expand inside the stent 82 (Figure 7D) and delivery is complete. The position of the wireform 90 relative to the tissue sleeve 88 constrains the tissue in such a way that the tissue sleeve is formed into valve leaflets. Conversely, the wireform 90 may have tissue leaflets already mounted to it and the tissue sleeve 88 is used solely to prevent perivalvular leak.

Figures 8A-D show an embodiment 100 that does not use a wireform. Rather device 100 comprises two separate components: a stent 102 and a valve assembly 104. The valve assembly 104 includes a first anchor ring 106, a second anchor ring 108, and a tissue sleeve 110 between the two anchor rings. The first anchor ring 106 anchors the valve assembly 104 in place to allow the tissue to be inverted.

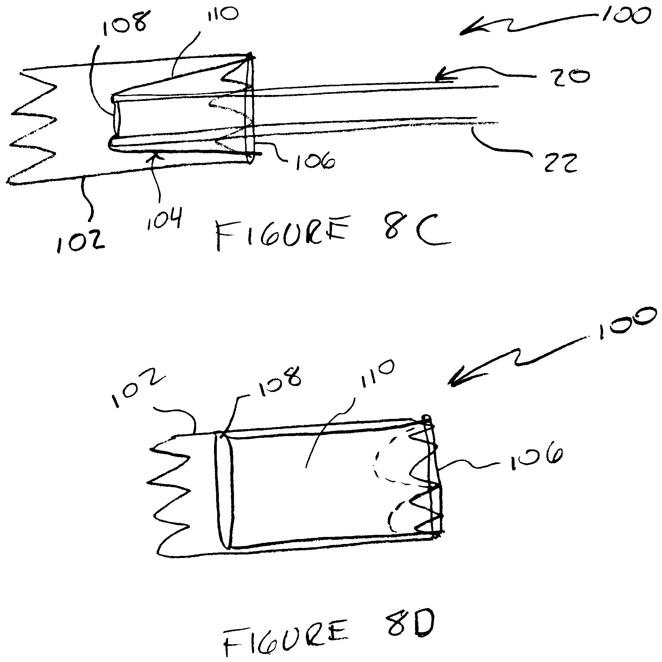

The delivery sequence for device 100 is as follows: As seen in Figure 8A, a stent 102 has been placed at the native valve site. The delivery catheter 20 is advanced until the distal end of the catheter is near of the proximal end of the stent 102. The sheath 22 of the delivery catheter 20 is then retracted releasing the ring 106. (Figure 8B) The ring expands just outside, or just inside of the stent 102. The delivery catheter 22 is advanced into the stent 102, causing the tissue sleeve 110 to invert. (Figure 8C) The second ring 108 is then released from the sheath 22 and allowed to expand inside the stent 102 (Figure 8D) and delivery is complete. The tissue sleeve 110 may have attachment points at discrete multiple locations 112 around the circumference of the ring 106 in order to define the commissural points of the prosthetic tissue valve.

Though expanding the first ring 106 on the aortic (proximal) side of the stent 102 may be advantageous in order to establish initial alignment, one could avoid the inversion step by deploying the first anchor ring 106 on the ventricular, or distal side of the sent 102 and then further retracting the sheath 22 until the second ring 108 is released and allowed to expand near the aortic side of the stent 102.

Although the invention has been described in terms of particular embodiments and applications, one of ordinary skill in the art, in light of this teaching, can generate additional embodiments and modifications without departing from or exceeding the scope of the claimed invention. Accordingly, it is to be understood that the drawings and descriptions herein are proffered by way of example to facilitate comprehension of the invention and should not be construed to limit the scope thereof.

## Claims

1. A device for replacing a native valve comprising:
a stent (102);
a tissue sleeve (110); and,
an anchoring mechanism usable to secure said tissue sleeve within said stent, said anchoring mechanism comprising a ring (106) attached to a first end of said tissue sleeve, **characterized in that** said anchoring mechanism comprises a second ring (108) attached to an opposite side of said tissue sleeve;
wherein, in a configuration inside a delivery catheter, said anchoring mechanism is not located within said stent; and
wherein, in a deployed configuration, said tissue sleeve is located within said stent.

2. The device of claim 1 wherein in said deployed configuration, said tissue sleeve is inverted relative to said configuration inside said delivery catheter.

3. The device of claim 1 wherein said tissue sleeve is connected to said stent at a first end and connected to said anchoring mechanism at a second end, opposite said first end.

4. The device of claim 1 wherein said anchoring mechanism comprises a wireform.

5. The device of claim 1 wherein said anchoring mechanism comprises a ring and a wireform.

6. The device of claim 1 wherein said tissue sleeve comprises pinch points that result in the formation of valve leaflets when implanted.

7. A valve assembly for implantation within a stent comprising:
a tissue sleeve (110);
an anchoring mechanism for securing said tissue sleeve within a stent, said anchoring mechanism comprising a first expandable ring (106) at one end of said tissue sleeve, **characterized in that** said anchoring mechanism comprises a second expandable ring (108) at another end of said tissue sleeve;
wherein said tissue sleeve is connected to said anchoring mechanism.

8. The valve assembly of claim 7 wherein said tissue sleeve is attached to a proximal end of said stent.

9. The valve assembly of claim 7 wherein said anchoring mechanism comprises a wireform.

10. The valve assembly of claim 7 wherein said tissue sleeve forms valve leaflets when said at least one anchoring mechanism is expanded.

11. The valve assembly of claim 7 wherein said tissue sleeve comprises valve leaflets that become functional after said tissue sleeve is inverted.

## Patentansprüche

1. Vorrichtung zum Ersetzen einer nativen Herzklappe, umfassend:
Stent (102);
Gewebehülse (110); und
Verankerungsmechanismus, der sich verwenden lässt, um die Gewebehülse innerhalb des Stents zu sichern;
wobei der Verankerungsmechanismus einen Ring (106) umfasst, der an einem ersten Ende der Gewebehülse befestigt ist, **dadurch gekennzeichnet, dass** der Verankerungsmechanismus einen zweiten Ring (108) umfasst, der an einer entgegengestzten Seite der Gewebehülse befestigt ist;
wobei sich der Verankerungsmechanismus in einer Konfiguration innerhalb eines Einführkatheters nicht innerhalb des Stents befindet, und
wobei sich die Gewebehülse in einer Einsatzkonfiguration innerhalb des Stents befindet.

2. Vorrichtung gemäß Anspruch 1, wobei die Gewebehülse in der Einsatzkonfiguration in Bezug auf die Konfiguration innerhalb des Einführkatheters invertiert ist.

3. Vorrichtung gemäß Anspruch 1, wobei die Gewebehülse an einem ersten Ende mit dem Stent und an einem zweiten Ende, welches dem ersten Ende entgegengesetzt ist, mit dem Verankerungsmechanismus verbunden ist.

4. Vorrichtung gemäß Anspruch 1, wobei der Verankerungsmechanismus eine Drahtform umfasst.

5. Vorrichtung gemäß Anspruch 1, wobei der Verankerungsmechanismus einen Ring und eine Drahtform umfasst.

6. Vorrichtung gemäß Anspruch 1, wobei die Gewebehülse Quetschpunkte umfasst, die bei Implantierung in der Bildung von Klappensegeln resultieren.

7. Herzklappenanordnung zur Implantierung innerhalb eines Stents, umfassend:
Gewebehülse (110),
Verankerungsmechanismus zur Sicherung der Gewebehülse innerhalb eines Stents, wobei der Verankerungsmechnismus einen ersten expandierbaren Ring (106) an einem Ende der Gewebehülse umfasst, **dadurch gekennzeichnet, dass** der Verankerungsmechanismus an einem anderen Ende der Gewebehülse einen zweiten expandierbaren Ring (108) umfasst;
wobei die Gewebehülse mit dem Verankerungsmechanismus verbunden ist.

8. Herzklappenanordnung gemäß Anspruch 7, wobei die Gewebehülse an einem proximalen Ende des Stents befestigt ist.

9. Herzklappenanordnung gemäß Anspruch 7, wobei der Verankerungsmechanismus eine Drahtform umfasst.

10. Herzklappenanordnung gemäß Anspruch 7, wobei die Gewebehülse Klappensegel bildet, wenn der mindestens eine Verankerungsmechanismus expandiert ist.

11. Herzklappenanordnung gemäß Anspruch 7, wobei die Gewebehülse Klappensegel umfasst, die nach Inversion der Gewebehülse funktionsfähig werden.

## Revendications

1. Dispositif destiné à remplacer une valve native comprenant :
une endoprothèse (102) ;
un manchon tissulaire (110) ; et,
un mécanisme d'ancrage utilisable pour fixer ledit manchon tissulaire dans ladite endoprothèse, ledit mécanisme d'ancrage comprenant un anneau (106) attaché à une première extrémité dudit manchon tissulaire, **caractérisé en ce que** le mécanisme d'ancrage comprend un deuxième anneau (108) attaché à un côté opposé dudit manchon tissulaire ;
dans lequel, dans une configuration à l'intérieur d'un cathéter de mise en place, ledit mécanisme d'ancrage n'est pas situé dans ladite endoprothèse ; et
dans lequel, dans une configuration déployée, ledit manchon tissulaire est situé dans ladite endoprothèse.

2. Dispositif selon la revendication 1 dans lequel dans ladite configuration déployée, ledit manchon tissulaire est inversé par rapport à ladite configuration à l'intérieur dudit cathéter de mise en place.

3. Dispositif selon la revendication 1 dans lequel ledit manchon tissulaire est connecté à ladite endoprothèse au niveau d'une première extrémité et connecté audit mécanisme d'ancrage au niveau d'une deuxième extrémité, opposée à ladite première extrémité.

4. Dispositif selon la revendication 1 dans lequel ledit mécanisme d'ancrage comprend une forme de fil.

5. Dispositif selon la revendication 1 dans lequel ledit mécanisme d'ancrage comprend un anneau et une forme de fil.

6. Dispositif selon la revendication 1 dans lequel ledit manchon tissulaire comprend des points de pincement qui résulte de la formation des valvules lorsqu'elles sont implantées.

7. Ensemble de valve pour une implantation dans une endoprothèse comprenant :
un manchon tissulaire (110) ;
un mécanisme d'ancrage destiné à fixer ledit manchon tissulaire dans une endoprothèse, ledit mécanisme d'ancrage comprenant un premier panneau extensible (106) à une première extrémité dudit manchon tissulaire, **caractérisé en ce que** ledit mécanisme d'ancrage comprend un deuxième anneau extensible (108) à une autre extrémité dudit manchon tissulaire ;
dans lequel ledit manchon tissulaire est connecté audit mécanisme d'ancrage.

8. Ensemble de valve selon la revendication 7 dans lequel ledit manchon tissulaire est attaché à une extrémité proximale de ladite endoprothèse.

9. Ensemble de valve selon la revendication 7 dans lequel ledit mécanisme d'ancrage comprend une forme de fil.

10. Ensemble de valve selon la revendication 7 dans lequel ledit manchon tissulaire forme des valvules lorsque ledit au moins un mécanisme d'ancrage est étendu.

11. Ensemble de valve selon la revendication 7 dans lequel ledit manchon tissulaire comprend des valvules qui deviennent fonctionnelles après que ledit manchon tissulaire a été inversé.
